# EUROPÄISCHE PATENTSCHRIFT

(11) **EP 1 932 472 B1**
(45) Veröffentlichungstag und Bekanntmachung des Hinweises auf die Patenterteilung: **08.02.2012**
(21) Anmeldenummer: 07021847.4
(22) Anmeldetag: 09.11.2007
(51) Int. Cl.: A61B 17/70, A61B 17/86, A61B 5/06, A61B 5/103

(54) **Medizinische Einrichtung zur Lagebestimmung von intrakorporalen Implantaten**
Medical device for determining the position of intracorporal implants
Dispositif médical destiné à la détermination spatiale d'implants intracorporels

(30) Priorität: 13.12.2006 DE 102006059226
(43) Veröffentlichungstag der Anmeldung: 18.06.2008
(73) Patentinhaber: Wittenstein AG, 97999 Igersheim (DE)
(72) Erfinder: Stauch, Roman, 97959 Assamstadt (DE); Wrobel, Miroslaw, 97753 Karlstadt (DE)
(74) Vertreter: Weiss, Peter

(56) Entgegenhaltungen:
- EP-A2- 0 135 804
- US-A1- 2006 276 803

## Beschreibung

Die Erfindung betrifft eine medizinische Einrichtung nach dem Oberbegriff des Anspruchs 1.

In diesem Zusammenhang wird auf die EP 0 135 804 A2 hingewiesen. Dort ist eine Vorrichtung zum Auffinden von Querbohrungen intramedullärer Implantate offenbart, wobei durch eine Magnetfeldmesseinrichtung in Gestalt einer Sonde eingesetzt wird.

Daneben wird die US 2006/0276803 A1 aufgezeigt, welche ein Wirbelsäulenimplantat mit einer elektrischen Steuerung offenbart.

Ein Implantat ist ein bei der Human- oder Veterinarmedizin in den jeweiligen Körper eingepflanztes künstliches Material, welches permanent oder zumindest für einen langen Zeitraum dort verbleiben soll. Hierbei sollen die medizinischen Implantate die Körperfunktionen unterstützen oder ersetzen.

Dank modernster Technologien gibt es mittlerweile chirurgische Implantate auch aus Kunststoff, Keramik, Kohlefasern oder dgl., sodass auch diese Materialien vielfach bei sogenannten. Fixationssystemen, Marknägeln, Platten und dgl. eingesetzt werden, bei denen eine Art von Ösenschrauben, genannt Pedikelschrauben zum Einsatz kommen, die z. B. im wesentlichen aus Kunststoff bestehen können und durch deren jeweilige Ösen die Fixationsstäbe gesteckt werden. Diese Fixationssysteme werden z. B. bei Wirbelfrakturen eingesetzt, die durch Stürze, Osteoporose, Tumore etc. verursacht sein können.

Nach der konventionellen Operationsmethode werden zur Einbringung der Pedikelschrauben und Fixationsstäbe die Muskeln großflächig von der Wirbelsäule abgelöst, wobei von den Pedikelschrauben jeweils eine zu beiden Seiten des Rückenmarkkanals eines Wirbelkörpers der Wirbelsäule in diesen geschraubt und die Fixationsstäbe von oben nach unten durch die Ösen der Pedikelschrauben gesteckt werden.

Bei jungen Menschen werden die Fixationsstäbe nach ca. einem 3/4 Jahr entfernt, da sonst die Gefahr besteht, dass die Schrauben/Stäbe mit zunehmender Mobilität des Patienten brechen könnten.

Bei älteren Menschen mit z. B. osteoporotischen Schäden (Frakturen, Sinterungen) an der Wirbelsäule wird vielfach bei einer Behandlung mit einem Stabsystem (Fixateur interne, ggf. in Verbindung mit Knochenzement) oder einem Plattensystem nach dem Fixateurprinzip in der Regel verbleibend fusioniert. Jedoch besteht hierbei die Gefahr eines "Durchschneidens" der Schrauben in den Bandscheibenraum wegen des verminderten Knochenwiderstands bei Osteoporose.

Demgegenüber erfolgt bei der sogenannten "Minimalinvasiven Methode" kein Freilegen der Wirbelsäule, sondern die Behandlung erfolgt lediglich durch kleine Schnitte von außen durch die Haut. Unter diese Methode fällt z. B. die Vertrebroplastie, bei der Knochenzement ohne vorausgehende Wiederaufrichtung der Wirbelkörper in diese gespritzt wird, oder die Kyphoplastie, bei der eingebrochene Wirbel mit Hilfe eines aufblasbaren Ballons und sodann eingespritztem Biozement wieder aufgerichtet werden. Dagegen ist das Einbringen von Fixationsstäben in der Frakturbehandlung von Wirbelkörpern durch ein minimalinvasives Verfahren bislang nicht möglich gewesen.

Hier setzt die Erfindung ein, der die Aufgabe zugrunde liegt, eine Möglichkeit zu schaffen, die Lage von Führungs- oder Verriegelungsbohrungen aus Kunststoff, Keramik oder dgl. bestehenden Implantaten in einfacher und sicherer Art und Weise bestimmen zu können, ohne dass dafür größere Operationseingriffe erforderlich sind.

Diese Aufgabe wird erfindungsgemäß durch den kennzeichnenden Teil des Anspruchs 1 gelöst.

Nach Maßgabe der Erfindung dient bei der Impedanzmessung als Messgröße zur Lagebestimmung die Phasenverschiebung zwischen Strom und Spannung, die in Abhängigkeit zum Abstand der intrakorporalen metallischen Sonde und dem magnetisch ortbaren Teil des Implantats zueinander auftritt, d. h., ist diese klein, dann bedeutet dies geringer Abstand und ist diese groß, dann bedeutet dies großer Abstand. Daraus ergibt sich: Die Größe der Impedanz ist eine Funktion des Abstands zwischen Sonde und Implantat.

Die Kombination von Impedanzmessung und Magnetfeldmessung hat den Vorteil, dass die Impedanz-Messeinrichtung für eine grobe und die Einrichtung zur Erzeugung und Ankopplung eines Magnetfeldes für eine genaue Lagebestimmung eingesetzt werden kann, wobei unter grobe Lagebestimmung die erste Annäherung der Sonde an das Implantat zu verstehen ist, wogegen dann der Restweg der Sonde bis zu dem oder sogar in das Implantat durch die genaue Lagebestimmung mit Hilfe der Einrichtung zur Erzeugung und Ankopplung eines Magnetfeldes erfolgt.

Mit anderen Worten im Vergleich mit der Beleuchtung eines Kraftfahrzeuges: "Fernlicht" für die grobe Positionierung durch Annäherung über die Impedanzmessung, wobei die Gewebezusammensetzung hierbei auch messbar ist, d. h., welches Gewebe gerade passiert wird und welche Vitalität dieses aufweist. Sobald dann hierbei eine relative Annäherung an das Implantat erreicht ist, wird auf "Abblendlicht" bzw. auf das Magnetfeld zur genaueren Positionierung umgeschaltet.

Falls mehrere Implantate z. B. hintereinander vorhanden sind, ist es zweckmäßig, die Impedanz-Messeinrichtung und die Einrichtung für das Magnetfeld alternierend schaltbar auszulegen, d. h., die grobe und die genaue Lagebestimmung erfolgt je fortschreitendem Implantat von neuem.

Ferner ist es möglich, die Impedanz-Messeinrichtung und die Einrichtung für das Magnetfeld derart zu schalten, dass sie gleichzeitig aktiv sind, was die Sondenführung verbessert.

Der wesentliche Vorteil der erfindungsgemäßen medizinischen Einrichtung liegt in der Verwendung bei der minimalinvasiven Chirurgie, denn dadurch müssen z. B. ausgedehnte Gewebe- und/oder Muskelbereiche nicht mehr abgelöst werden, wodurch eine Verminderung des Blutverlustes, eine geringere Muskelnervenschädigung, die Erhaltung der Propriorezeption, eine geringere Narbenbildung an Haut- und Muskelbereich und damit eine Reduzierung der Liegezeiten sowie eine frühere Mobilisierung der Patienten möglich ist. Schließlich kann auch die Röntgen-Strahlenbelastung durch den Einsatz der Magnetfeldtechnologie für den Arzt und den Patienten während der intraoparativen Applikation reduziert werden.

Die erfindungsgemäße Einrichtung ist insbesondere in der minimalinvasiven Chirurgie bei einem Wirbelsäulenfixationssystem mit metallischen Stäben als Stabilisatoren und mit sogenannten magnetisch ortbaren Pedikelschrauben einsetzbar, und zwar dienen hier die Stäbe als intrakorporalbare Sonden mit denen die Impedanz-Messeinrichtung und die Einrichtung für das Magnetfeld in Verbindung stehen, wobei der zusätzliche Anschluss der Impedanz-Messeinrichtung mit den magnetisch ortbaren Pedikelschrauben in elektrisch leitender Verbindung steht.

Es ist zweckmäßig, dass die intrakorporale Sonde stabförmig ausgebildet ist und an ihrem freien Handende ein Element zur Erzeugung eines räumlichen, möglich konstanten Magnetfeldes (Solenoid) aufweist, wobei dessen Befestigung an der Sonde durch ein Gewinde, Schnellverschluss od. dgl. erfolgen sollte, damit dieser am Ende einer Operation wieder entfernt werden kann. Gleichzeitig muss aber sichergestellt sein, dass sich diese Gewindeverbindung beim Manipulieren während einer Operation nicht lösen kann, was insbesondere nicht bei einer Drehung der Sonde nach links und nach rechts gegeben sein darf, weswegen hier ein geeignetes Feingewinde zu bevorzugen ist.

Das Arbeiten mit der erfindungsgemäßen Einrichtung erfolgt in der Weise, dass zuerst mit der Sonde bei deren intrakorporalen Einführung eine grobe Lagebestimmung durchgeführt wird, und zwar indem mit Hilfe der Impedanz-Messeinrichtung die Phasenverschiebung zwischen Strom und Spannung in Abhängigkeit zum Abstand der Sonde und dem Implantat zueinander gemessen wird, während kurz vor dem Erreichen des Implantats durch die Sonde eine genaue Lagebestimmung erfolgt, die dann mit Hilfe der Einrichtung für das Magnetfeld in bekannter Weise durchgeführt wird.

Weitere Merkmale und Vorteile der vorliegenden Erfindung ergeben sich aus der nachfolgenden Beschreibung eines Ausführungsbeispiels in Verbindung mit der Zeichnung; es zeigen:
- Fig.1: eine Seitenansicht eines schematisch dargestellten Abschnitts einer Wirbelsäule, an der erfindungsgemäß drei Wirbelkörper miteinander verbunden, d. h. stabilisiert werden sollen und
- Fig. 2: eine Draufsicht auf eine schematisch dargestellte, so genannte Pedikelschraube aus Kunststoff mit eingesetzter Metallbuchse wie sie bei dem Beispiel in Fig. 1 zur Stabilisierung der Wirbelkörper verwendet wird.

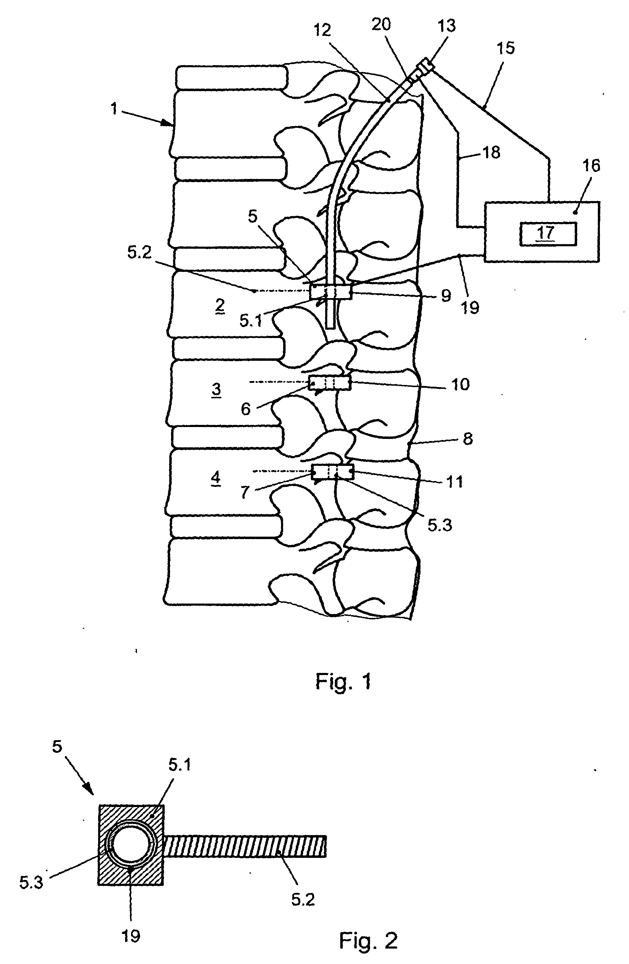

Im Einzelnen zeigt die Fig. 1 einen seitlichen Abschnitt einer Wirbelsäule 1, in deren drei Wirbelkörper 2 - 4 jeweils so genannte Pedikelschrauben 5 - 7 geschraubt sind, wozu die mit 8 bezeichnete Haut lediglich einen relativ kleinen Schnitt erhält, - angedeutet bei 9 -11- . Die einzelnen Pedikelschrauben 5 - 7 bestehen im wesentlichen aus Kunststoff und - am Beispiel der ersten Schraube 5 gesehen - aus einer Öse 5.1, einem Gewindeschaft 5.2 und einer in die Öse 5.1 eingesetzten Metallbuchse 5.3 (Fig. 2), welche aus Titan besteht, wobei Chrom- und Kobalt-Nickellegierungen ebenfalls möglich sind.

Neben dem Beispiel in Fig. 2 erstreckt sich die vorliegende Erfindung auch auf andere chirurgische Implantate, wie Marknägel, Ankerplatten, Schrauben, Implantate, die mit einem Knochen verbunden werden müssen usw. Alle diese Implantate, welche aus Kunststoff, Keramik oder dgl. hergestellt werden können, haben eines gemeinsam, nämlich sie müssen magnetisch ortbar ausgebildet sein, d. h., sie müssen mindestens einen elektrisch leitfähigen Marker aufweisen, wie einen metallischen Einsatz, metallische Fasern, Metallpulver oder dgl.

Die drei Wirbelkörper 2 - 4 gemäß Fig. 1 sollen miteinander verbunden, d. h. stabilisiert werden, wozu nach dem minimalinvasiven Verfahren ein elektrisch leitender Fixationsstab 12 dient, der am seinem Kopfende einen Solenoid 13 besitzt, der über eine elektrische Leitung 15 mit einer Impedanz-Messeinrichtung 16 mit einer optischen und/oder akustischen Anzeigeeinrichtung (Display) 17 verbunden ist. Gleichzeitig ist die Impedanz-Messeinrichtung 16 über die Leitung 18 mit dem Fixationsstab 12 verbunden und über einen Anschluss 19 mit der Buchse 5.3 der Pedikelschraube 5.

Das minimalinvasive Setzen der Pedikelschrauben 5 - 7 und des Fixationsstabes 12 geschieht wie folgt, wobei in Fig. 1 nur eine Hälfte der Schraubenanordnung gezeigt ist, denn praktisch werden zu beiden Seiten des Rückenmarkkanals der betroffenen Wirbelkörper 2 - 4 eine Schraube gesetzt, sodass zwei Reihen Schrauben mit je einem Fixationsstab 12 vorhanden sind, von denen jedoch nur eine Reihe in Fig. 1 zu sehen ist.

Zum Eindrehen der Pedikelschrauben 5 - 7 werden in der Haut 8 entsprechend kurze Schnitte 9 - 11 eingebracht und die Pedikelschrauben 5 - 7 nach einem Vorbohren mit ihrem jeweiligen Gewindeschaft (Beispiel: 5 → 5.2) in die Wirbelkörper 2 - 4 (Beispiel: 2 ) derart eingeschraubt, dass ihre Ösen (Beispiel: 5.1) waagrecht stehen. Hierbei ist zu beachten, dass die Schrauben 5 - 7 durch den jeweiligen Muskel hindurch gesetzt werden, d. h. hierzu soll nach Möglichkeit eine Längsteilung bzw. Verdrängung des Muskels erfolgen.

Zum minimalinvasiven Einbringen des Fixationsstabes 12 durch die metallischen Buchsen der sonst aus Kunststoff bestehenden Pedikelschrauben 5 - 7 soll der Stab ebenfalls nach Möglichkeit durch den Muskel geschoben werden, wobei zu Beginn die Haut 8 durch einen nicht näher bezeichneten Schnitt durchdrungen werden muss.

Als Zielhilfe für den Fixationsstab 12 dient zu Beginn die Impedanzmessung, bei der als Messgröße die Phasenverschiebung zwischen Strom und Spannung dient, welche über die Anzeigeeinrichtung 17 ablesbar und/oder akustisch wahrnehmbar ist, d. h. je kleiner diese Phasenverschiebung ist, desto näher befindet sich die Spitze des Fixationsstabes 12 an der metallischen Buchse 5.3 der Öse 5.2. Wenn dieses "Δ klein" einen bestimmten Nahbereich erreicht hat, wird auf das Magnetfeld, d. h. auf bspw. den Solenoid (stromdurchflossener Stabmagnet) umgeschaltet, da mit diesem der Fixationsstab 12 genauer und somit besser durch die Buchse 5.3 geführt werden kann.

Das Weiterführen des Fixationsstabes 12 durch die Buchsen der beiden weiteren Pedikelschrauben 6 und 7 erfolgt analog, wobei dann die Leitung 19 jeweils mit den entsprechenden Buchsen dieser Schrauben verbunden werden muss. Hierbei können zwei Möglichkeiten gewählt werden, und zwar erst die Impedanzmethode als Groborientierung und dann für die genauere Annäherung und das Durchführen durch die Ösen die Einrichtung zur Erzeugung und Ankoppelung des Magnetfeldes oder beides.

Die Befestigung des Solenoid 13 an dem Fixationsstab 12 als Sonde ist durch ein Feingewinde, einen Schnellverschluss od. dgl. bewerkstelligt, und zwar damit diese Einheit am Ende des Eingriffs wieder entfernt werden kann.

Ferner besitzt der in der Wirbelsäule 1 verbleibende Abschnitt des Fixationsstabes 12 an seinem Außenumfang eine Kerbe 20, damit dieser Abschnitt zu gegebener Zeit mit Hilfe eines geeigneten Extraktionsinstruments wieder entfernt werden kann.

Insgesamt ist mit der vorliegenden Erfindung somit ein weiterer Meilenstein in der minimalinvasiven Chirurgie zum Wohle der Patienten gefunden worden.

### Bezugszeichenliste

| | | | | | |
|---|---|---|---|---|---|
| 1 | Wirbelsäule | | | | |
| 2 | Wirbelkörper | | | | |
| 3 | Wirbelkörper | | | | |
| 4 | Wirbelkörper | | | | |
| 5 | Pedikelschraube | 5.1 | Öse | 5.2 | Gewindeschaft |
| 6 | Pedikelschraube | | | 5.3 | Metallbuchse |
| 7 | Pedikelschraube | | | | |
| 8 | Haut | | | | |
| 9 | Schnitt | | | | |
| 10 | Schnitt | | | | |
| 11 | Schnitt | | | | |
| 12 | Sonde/Fixationsstab | | | | |
| 13 | Solenoid | | | | |
| 14 | | | | | |
| 15 | Leitung | | | | |
| 16 | Impedanz-Messeinricht. | | | | |
| 17 | Anzeigeeinrichtung | | | | |
| 18 | Leitung | | | | |
| 19 | Anschluss | | | | |
| 20 | Kerbe | | | | |
| 21 | | | | | |
| 22 | | | | | |
| 23 | | | | | |
| 24 | | | | | |
| 25 | | | | | |
| 26 | | | | | |
| 27 | | | | | |
| 28 | | | | | |
| 29 | | | | | |
| 30 | | | | | |
| 31 | | | | | |
| 32 | | | | | |
| 33 | | | | | |

## Patentansprüche

1. Medizinische Einrichtung zur Lagebestimmung von intrakorporalen Implantaten wie Fixationssysteme, Ankerplatten oder dgl., mit einer Einrichtung zur Erzeugung und Ankopplung eines Magnetfeldes (13) zur Lagebestimmung von Implantaten,
**dadurch gekennzeichnet,**
**dass** diese mindestens eine Impedanz-Messeinrichtung (16) und die Einrichtung zur Erzeugung und Ankopplung eines Magnetfeldes (13) umfasst, die beide mit mindestens einer intrakorporalen Sonde (12) in Verbindung stehen, wobei die Impedanz-Messeinrichtung (16) zusätzlich noch mit mindestens einem Anschluss (19) für eine Verbindung mit einem leitfähigen Teil des Implantates (5) ausgestattet ist.

2. Medizinische Einrichtung nach Anspruch 1, **dadurch gekennzeichnet, dass** zur Lagebestimmung bei der Impedanzmessung als Messgröße die Phasenverschiebung zwischen Strom und Spannung dient, die in Abhängigkeit zum Abstand der intrakorporalen Sonde (12) und dem magnetischen Teil des Implantates (5) zueinander auftritt.

3. Medizinische Einrichtung nach einem der Ansprüche 1 oder 2, **dadurch gekennzeichnet, dass** die Impedanz-Messeinrichtung (16) für eine grobe und die Einrichtung zur Erzeugung und Ankopplung eines Magnetfeldes (13) für eine genaue Lagebestimmung dienen.

4. Medizinische Einrichtung nach Anspruch 3, **dadurch gekennzeichnet, dass** die Impedanz- Messeinrichtung (16) und die Einrichtung zur Erzeugung und Ankopplung eines Magnetfeldes (13) alternierend schaltbar sind.

5. Medizinische Einrichtung nach Anspruch 4, **dadurch gekennzeichnet, dass** die Impedanz-Messeinrichtung (16) und die Einrichtung zur Erzeugung und Ankopplung eines Magnetfeldes (13) derart geschaltet sind, dass sie gleichzeitig aktiv sind.

6. Medizinische Einrichtung nach einem der Ansprüche 1 bis 5, **dadurch gekennzeichnet, dass** die intrakorporale Sonde (12) stabförmig ausgebildet ist und an ihrem freien Handende ein Element zur Erzeugung eines räumlich, möglichst konstanten Magnetfeldes, insbesondere ein Solenoid (13), (14) aufweist.

7. Medizinische Einrichtung nach Anspruch 6, **dadurch gekennzeichnet, dass** die Befestigung des Elementes, insbesondere des Solenoiden (13) an der Sonde (12) durch ein Gewinde, insbesondere eine Feingewinde erfolgt.

8. Medizinische Einrichtung nach Anspruch 6, **dadurch gekennzeichnet, dass** ein intrakorporales Teil der stabförmigen Sonde (12) zumindest an einem seiner Enden eine Vertiefung wie Kerbe (20) für ein Ansetzen eines Extraktionsinstrumentes aufweist.

## Claims

1. A medical device for determining the position of intracorporeal implants such as fixation systems, anchor plates or the like, having a device for generating and coupling a magnetic field (13) for determining the position of implants,
**characterised in that** it comprises at least one impedance measuring device (16) and the device for generating and coupling a magnetic field (13), both of which are connected to at least one intracorporeal probe (12), wherein the impedance measuring device (18) is additionally provided with at least one connection (19) for a connection to a conductive part of the implant (5).

2. A medical device according to claim 1, **characterised in that** the phase shift between current and voltage serves as a measurand for determining position upon impedance measurement, which phase shift occurs dependent on the spacing of the intracorporeal probe (12) and the magnetic part of the implant (5) from one another.

3. A medical device according to one of claims 1 and 2, **characterised in that** the impedance measuring device (16) is used for a rough determination of position and the device for generating and coupling a magnetic field (13) is used for an exact determination of position.

4. A medical device according to claim 3, **characterised in that** the impedance measuring device (16) and the device for generating and coupling a magnetic field (13) are alternately switchable.

5. A medical device according to claim 4, **characterised in that** the impedance measuring device (16) and the device for generating and coupling a magnetic field (13) are switched in such a manner that they are active at the same time.

6. A medical device according to any one of claims 1 to 5, **characterised in that** the intracorporeal probe (12) is rod-shaped and at its free hand-operated end has an element for generating a three-dimensional magnetic field which is as constant as possible, in particular a solenoid (13), (14).

7. A medical device according to claim 6, **characterised in that** securing of the element, in particular of the solenoid (13), to the probe (12) takes place by means of a thread, in particular a fine-pitch thread.

8. A medical device according to claim 6, **characterised in that** an intracorporeal part of the rod-shaped probe (12) has, at least at one of its ends, a depression such as a groove (20) for an application of an extraction instrument.

## Revendications

1. Dispositif médical destiné à la détermination spatiale d'implants intracorporels, tels que systèmes de fixation, plaques d'ancrage ou similaires, avec un dispositif de génération et de couplage d'un champ magnétique (13) destiné à la détermination spatiale d'implants,
**caractérisé par le fait**
**qu'**il comporte au moins un dispositif de mesure d'impédance (16) et le dispositif de génération et de couplage d'un champ magnétique (13) qui communiquent l'un avec l'autre par au moins une sonde intracorporelle (12), le dispositif de mesure d'impédance (16) étant, en outre, encore équipé d'au moins une connexion (19) pour un raccordement à une partie conductrice de l'implant (5).

2. Dispositif médical selon la revendication 1, **caractérisé par le fait qu'**à la détermination spatiale lors de la mesure d'impédance sert de grandeur de mesure le déphasage entre le courant et la tension qui se produit en fonction de la distance entre la sonde intracorporelle (12) et la partie magnétique de l'implant (5).

3. Dispositif médical selon l'une des revendications 1 ou 2, **caractérisé par le fait que** le dispositif de mesure d'impédance (16) sert à une détermination spatiale approximative et le dispositif de génération et de couplage d'un champ magnétique (13) sert à une détermination spatiale précise.

4. Dispositif médical selon la revendication 3, **caractérisé par le fait que** le dispositif de mesure d'impédance (16) et le dispositif de génération et de couplage d'un champ magnétique (13) peuvent être commutés en alternance

5. Dispositif médical selon la revendication 4, **caractérisé par le fait que** le dispositif de mesure d'impédance (16) et le dispositif de génération et de couplage d'un champ magnétique (13) sont connectés de sorte qu'ils soient actif en même temps.

6. Dispositif médical selon l'une des revendications 1 à 5, **caractérisé par le fait que** la sonde intracorporelle (12) est réalisée sous forme de barre et présente, à son extrémité de main libre, un élément destiné à générer un champ magnétique spatialement le plus stable possible, en particulier un solénoïde (13), (14).

7. Dispositif médical selon la revendication 6, **caractérisé par le fait que** la fixation de l'élément, en particulier du solénoïde (13), à la sonde (12) a lieu par un filetage, en particulier un filetage fin.

8. Dispositif médical selon la revendication 6, **caractérisé par le fait qu'**une partie intracorporelle de la sonde en forme de barre (12) présente, au moins à l'une de ses extrémités, une cavité, telle qu'une encoche (20) pour le placement d'un instrument d'extraction.
